## Europäisches Patentamt

(⑩) **European Patent Office**

**Office européen des brevets**

(⑪) Publication number: **0 176 288**
**B1**

(⑫) **EUROPEAN PATENT SPECIFICATION**

(㊺) Date of publication of patent specification: **09.05.90**

(㉑) Application number: **85306500.1**

(㉒) Date of filing: **12.09.85**

(㊿) Int. Cl.⁵: **C 07 C 229/76,**
**C 07 C 227/00, C 07 B 59/00,**
**A 61 K 43/00**

(㊴) Aminocarboxylic acid complexes for the treatment of calcific tumors.

(㉚) Priority: **21.09.84 US 652702**
**05.08.85 US 762361**

(㊸) Date of publication of application:
**02.04.86 Bulletin 86/14**

(㊺) Publication of the grant of the patent:
**09.05.90 Bulletin 90/19**

(㊴) Designated Contracting States:
**BE CH DE FR GB IT LI NL SE**

(㊻) References cited:
**EP-A-0 083 129**
**DE-A-3 401 052**

**CHEMICAL ABSTRACTS, vol. 70, no. 19, 12 May 1969, Columbus, OH (US); R.E.O'MARA et al.: "Rare carth nuclides as potential agents for skeletal imaging", p. 64, no. 84881u**

The file contains technical information submitted after the application was filed and not included in this specification

(㊂) Proprietor: **THE DOW CHEMICAL COMPANY**
**2030 Dow Center Abbott Road P.O. Box 1967**
**Midland Michigan 48640-1967 (US)**

(�72) Inventor: **Simon, Jaime**
**Rt. 1, Box 120G**
**Angleton, TX 77515 (US)**
Inventor: **Wilson, David A.**
**229 San Saba**
**Richwood, TX 77531 (US)**
Inventor: **Volkert, Wynn A.**
**2203 Garden Drive**
**Columbia, MO 65212 (US)**
Inventor: **Troutner, David E.**
**402 Edgewood Avenue**
**Columbia, MO 65201 (US)**
Inventor: **Goeckeler, William F.**
**740 Demaret, Apt.**
**Columbia, MO 65202 (US)**

(㊄) Representative: **Burford, Anthony Frederick et al**
**W.H. Beck, Greener & Co. 7 Stone Buildings Lincoln's Inn**
**London WC2A 3SZ (GB)**

Courier Press, Leamington Spa, England.

EP 0 176 288 B1

(56) References cited:

CHEMICAL ABSTRACTS, vol. 76, no. 7, 14 Feb 1972, Columbus, OH (US); S.Y.CHOWDHARY et al.: "Formation and control of Samarium and ytterbium chelate complexes used as radiopharmaceuticals", p. 219, no. 37422j

CHEMICAL ABSTRACTS, vol. 84, no. 18, 03 May 1976, Columbus, OH (US); E.D.FILIPPOVA et al.: "Hydrate composition of rare earth element hydroxyethylethylenediamine-triacetates", p. 580, no. 129901h.

CHEMICAL ABSTRACTS, vol. 70, no. 21, 26 May 1969, Columbus, OH (US); E.BRUCHER et al.: "Central-ion exchange reactions of rare earth metal-aminopoly(acetic acid)complexes. II. Ion-exchange reactions of Lu3+ ions", p.279, no. 100133f

Rosoff et al (Int. J. App. Red. and Iso.(1963) 14, pages 129-135),"Distribution and Excretions of Radioactive Rare-Earth Components in Mice"

**Description**

Aminocarboxylic acids are known to chelate metal ions. Particularly stable chelates are formed with metals from the alkaline earth and transition metal series.

The development of a bone metastasis is a common and often catastrophic event for a cancer patient. The pain, pathological fractures, frequent neurological deficits and forced immobility caused by these metastatic lesions significantly decrease the quality of life for the cancer patient. The number of patients that contract metastatic disease is large since nearly 50 percent of all patients who contract breast, lung or prostate carcinoma will eventually develop bone metastases. This indicates the prevalence of the disease. Bone metastases are also seen in patients with carcinoma of the kidney, thyroid, bladder, cervix and other tumors, but collectively, these represent less than 20 percent of patients who develop bone metastases. Metastatic bone cancer is rarely life threatening and occasionally patients live for years following the discovery of the bone lesions. Initially, treatment goals center on relieving pain, reducing requirements for narcotic medication and increasing ambulation. Clearly, it is hoped that some of the cancers can be cured.

The use of radionuclides for treatment of cancer metastatic to the bone dates back to the early 1950's. It has been proposed to inject a radioactive particle-emitting nuclide in a suitable form for the treatment of calcific lesions. It is desirable that such nuclides be concentrated in the fast growing portion of the bone with minimal amounts reaching the soft tissue and normal bone. Radioactive phosphorus compounds (P-32 and P-33) have been proposed. However, the nuclear and biolocalization properties limit the use of these compounds. (Kaplan, E., et al, *Journal of Nuclear Medicine*, Vol. 1, No. 1, page 1, 1960); (U.S. Patent 3,965,254).

Another attempt has been made using phosphorus compounds containing a boron residue. The compounds were injected into the body (intravenously) and accumulated in the skeletal system. The patient was then irradiated with neutrons in order to activate the boron and give a therapeutic radiation dose. (U.S. Patent 4,399,817).

In the above-mentioned procedures, it is not possible to give therapeutic doses to the tumor without substantial damage to normal tissues. In many cases, especially for metastic bone lesions, the tumor has spread throughout the skeletal system and amputation or irradiation of a portion of the body is not practical. (*Seminars in Nuclear Medicine*, Vol. IX, No. 2, April, 1979).

The use of diphosphonate complexed with Re-186 has also been proposed. (Mathieu, L. et al, *Int. J. Applied Rad. & Isotopes*, Vol. 30, pp. 725—727, 1979; Weinenger, J., Ketring, A. R., et al, *Journal of Nuclear Medicine*, Vol. 24, No. 5, p. 125, 1983). However, the preparation and purification needed for this complex limits its utility and wide application. ·

Strontium-89 has also been proposed for patients with metastic bone lesions. However, the long half-life (50.4 days), high blood levels and low lesion to normal bone ratios limit the utility. (Firusian, N., Mellin, P., Schmidt, C. G., *The Journal of Urology*, Vol. 116, page 764, 1976; Schmidt, C. G., Firusian, N., *Int. J. Clin. Pharmacol.*, 93:199—205, 1974).

A palliative treatment of bone metastases has been reported which employed I-131 labelled α-amino-(3-iodo-4-hydroxybnenzylidene)diphosphonate (Eisenhut, M., *Journal of Nuclear Medicine*, , Vol. 25, No. 12, pp. 1356—1361, 1984). The use of radioiodine as a therapeutic radionuclide is less than desirable due to the well known tendency of iodide to localize in the thyroid. Eisenhut lists iodide as one of the possible metabolites of this compound. In addition, any I-131 left over from the iodination reaction and not separated in the washing procedure also constitutes a threat to the thyroid. Indeed Eisenhut finds 0.1 percent of the injected dose present in the thyroid 24 hours after injection. The high energy of the gamma ray emission from I-131 leads to inferior quality images.

O'Mara et al (*Journal of Nuclear Medicine, 10*, pp. 49—51, 1969) have prepared rare earth complexes of aminocarboxylic acids. They find good skeletal properties and propose their use as diagnostic skeletal agents. However, the utility of these agents for therapy is not mentioned. The amount of radionuclide which must reach the bone is far greater for therapeutic use than when it is employed for diagnostic purposes (eg. ~ 15 mCi (0.5GBq) injection of Tc-99m for imaging, ~ 75 mCi (3GBq) injection of Sm-153 for therapy of a 150-lb. (70Kg) man).

It has now been discovered that certain particle-emitting radionuclides when complexed with such aminocarboxylic acids can be effectively used as therapeutic agents in the treatment of bone cancer.

Particle-emitting radionuclides, e.g. Samarium-153, have been complexed with aminocarboxylic acid compounds wherein at least one of the amine hydrogens is replaced by a carboxyalkyl group. These complexes show promise in the treatment of calcific tumors in animals.

Therapeutically useful or effective complexes according to the present invention must, insofar as possible, fit certain criteria. While there are many aminocarboxylic acids, only a relative few fit the requirements of being therapeutically effective and then only in combination with certaion radionuclides. While the properties of the particular isotope of the radionuclide are important, the properties of the combination as the complex is the determining factor. The disadvantages of any one property may be overcome by the superiority of one or more of the properties of either ligand or isotope and their combination as the complex must be considered in toto.

The following is a discussion of those criteria which must be considered in choosing any particular combination of radioisotope and aminocarboxylic acid ligand. Certain combinations, due to one or more undesirable properties may not be therapeutically useful or effective, e.g. too much radiation localizing in the bone marrow.

There is a need, therefore, for a system possessing the following criteria by which it is possible to deliver therapeutic radiation doses to calcific tumors with minimal doses to soft tissue or normal bone.

Firstly, the complex must be taken up preferentially by the bone rather than by soft tissue. Most particularly, uptake in either liver or bone marrow is undesirable. The treatment should result in no long-term suppression of bone marrow.

Another important criterion is the ratio of the amount of complex taken up by the cancerous bone to that by normal bone. High ratios are preferred since it is desired to treat the cancerous bone while irradiating the normal bone as little as possible.

The complex should be cleared rapidly from non-osseous tissue to avoid unnecessary damage to such tissues, e.g. it should clear rapidly from the blood.

With respect to the complexed radionuclide, considerations of the nuclear properties are essential. First, the half-life must be sufficiently long to allow for localization in the bone without delivering excessive doses to non-target organs. When the complex is able to biolocalize rapidly, isotopes having shorter half-lives are useful. Isotopes with shorter half-lives provide an advantage since this permits the total dose to be given fractionally, allowing any non-target organs damaged by the radiation to recover between doses.

While for successful treatment of tumors the isotope must have sufficient particle emission, it is desirable, in order to quantify the localization of the complex, that the isotope also have sufficient gamma ray production that imaging is possible. The preferred gamma ray energy should be in the 100 to 200 keV $(16 \times 10^{-15}$ to $32 \times 10^{-15}$ joule) range although some isotopes having higher energies are potentially useful. The amount of gamma radiation should be sufficient to image, but not so great that the patient needs to be isolated to prevent him from becoming a source of radiation exposure to persons near him.

The proposed use for the complexes of this invention is in the therapeutic treatment of calcific tumors. These include primary tumors, where the skeletal system is the first site of involvement, and metastatic bone cancer where the neoplasm spreads from other primary sites, e.g. prostate, breast, into the skeletal system. This invention provides a method of alleviating pain by delivering a therapeutic radiation dose to the aforementioned calcific tumors. This invention also provides a means of reducing the size of or destroying the calcific tumors by delivering a therapeutic radiation dose to calcific tumors.

A therapeutically effective or useful particle-emitting radionuclide as defined herein is one capable of delivering a high enough localized ionization density to kill tumor cells and is capable of forming complexes with the ligands described herein. The effective amount may be administered as a single dose or in multiple doses over a longer period of time. Delivery of the complexed radionuclide to the tumor must be in sufficient amounts to alleviate pain and/or inhibit tumor growth and/or cause regression of the tumor.

The organic carboxylic acid derivatives which have been found useful in complexing the particle-emitting radionuclides are amino compounds wherein at least one amine hydrogen is replaced by a carboxyalkyl radical. Carboxylamines are suitably prepared according to the method disclosed in *Chelating Agents and Metal Chelates,* Ed. F. P. Dwyer, D. P. Mellor, Academic Press, NY (1964), pp. 285—286. Particle-emitting radionuclides, e.g. Samarium-153 (Sm-153), Ytterbium-175 (Yb-175), Lutetium-177 (Lu-177) and Gadolinium-159 (Gd-159), have been complexed with aminocarboxylic acids. We have now found that those aminocarboxylic acid complexes with rare earth metals possessing Log K values of from 13 to 20 fit the aforementioned criteria. K is an equilibrium ocnstant defined by

$$\frac{[M \cdot L]}{[M] \times [L]}$$

wherein the bracketed letters represent concentrations of metal (M), ligand (L) and complex (M·L). For chelation reactions this constant is called the stability constant and is a measure of the affinity of the complexing agent for a particular metal. Since protons compete with metal ions for the ligand, the stability constant is pH dependent. To describe the reaction at a specific pH, conditional stability constants can be calculated. (See Fritz, J. S. and Schenk, G. H., *Quantitative Analytical Chemistry,* Allyn and Bacon, Inc., Boston (1973)). For example, Sm-153- nitrilotriacetic acid (Sm-153-NTA), Log K = 11.3, has a high liver uptake and is thus unsuitable for therapeutic use. Furthermore, Sm-153-diethylene-triaminepentaacetic acid (Sm-153-DTPA), Log K = 22.3, has low skeletal uptake and thus is also considered to be unacceptable for use. Examples of appropriate aminocarboxylic acids and their Log K values are found in Martel, A. E. and Smith, R. M., *Critical Stability Constants,* Vol. 1, Plenum Press, New York and London (1974), and Sillen, L. G. and Martel, A. E., *Stability Constants of Metal-Ion Complexes,* Supplement Nos. 17 and 25, The Chemical Society, Burlington House, London (1964; 1977). Representative examples of aminocarboxylic acids which are useful in preparing the complexes employed in this invention are ethylenediaminecarboxylic acids such as ethylenediamineacetic acids. Preferred ethylenediamineacetic acids have at least one of the amine hydrogens replaced by a hydroxyalkyl radical containing 2 or 3 carbon atoms. Particularly preferred are hydroxyethylethylenediaminetriacetic acid (HEEDTA) and ethylenediaminetetraacetic acid (EDTA).

While Sm-153, Yb-175, Lu-177 and Gd-159 have been exemplified as the rare earth radionuclides employed with the complexing agents above, other rare earth radionuclides may also be complexed with the same aminocarboxylic acid derivatives as disclosed herein, e.g. Ho-166.

For the purpose of the present invention, therapeutically effective complexes described herein and physiologically acceptable salts thereof are considered equivalent. Physiologically acceptable salts refer to the acid addition salts of those bases which will form a salt with at least one acid group of the complex and which will not cause an adverse physiological effect when administered to an animal at dosages consistent with good pharmacological activity. Suitable bases include, for example, the alkali metal and alkaline earth metal hydroxide, carbonates, and bicarbonates such as sodium hydroxide, potassium hydroxide, calcium hydroxide, potassium carbonate, sodium bicarbonate, magnesium carbonate and the like, ammonia, primary, secondary and tertiary amines and the like. Physiologically acceptable salts of the present invention may be prepared by treating the complex having at least one acid group with an appropriate base.

Radionuclides can be produced in several ways. In a reactor, a nuclide is bombarded with neutrons to obtain a nuclide with additional neutrons in its nucleus.

$$\text{e.g. Sm-152} + \text{neutron} \rightarrow \text{Sm-153} + \text{gamma}$$

Another method of obtaining radioisotopes is by bombarding nuclides with linear accelerator or cyclotron-produced particles. Yet another way of obtaining radioisotopes is to isolate them from fission product mixtures. The method of obtaining the radionuclide is not critical to the present invention.

The Samarium used in the following examples was either natural $Sm_2O_3$ (99.9 percent from Spex Industries) or isotopically enriched (99.06 percent Sm-152) $Sm_2O_3$.

The Sm-153 used in this study was produced by neutron irradiation at the University of Missouri Research Reactor. Preliminary studies were carried out using Sm-153 produced by short (5 to 30 minutes) irradiations of natural $Sm_2O_3$, in the reactor's pneumatic tube system. The specific activity of Sm-153 produced by this method was 0.5—3.0 Ci/g (18 to 110 GBq/g).

The majority of this work was carried out using Sm-153 produced by irradiating 99.06 percent enriched $^{152}Sm_2O_3$ in the first row reflector at a neutron flux of $1 \times 10^{14}$ neutron/cm$^2$·sec. Irradiations were generally carried out for 50 to 60 hours, yielding a Sm-153 specific activity of 1000 to 1300 Ci/g ($37 \times 10^3$ to $48 \times 10^3$ GBq/g).

To irradiate $Sm_2O_3$ for production of Sm-153, the desired amount of target was first weighed into a quartz vial, the vial flame sealed under vacuum and welded into an aluminum can. The can was irradiated for the desired length of time, cooled for several hours and opened remotely in a hot cell. The quartz vial was removed and transferred to a glove box, crushed into a glass vial which was then sealed with a rubber septum and an aluminum crimp cap. One milliliter of 1—4 M HCl was then added to the vial via syringe to dissolve the $Sm_2O_3$. Once dissolved, the solution was diluted to the appropriate volume by addition of water. The solution was removed from the original dissolution vial which contains the chards of the crushed quartz vial, and transferred via syringe to a clean glass serum vial. This solution was then used for complex preparation. Similar procedures were used to prepare other radionuclides, e.g. Lu-177, Yb-175, Gd-159.

When aqueous solutions of metal ions are mixed with solutions containing complexing agents, such as those described in this invention, a complex between the metal ion and the ligand can be formed as shown by the equation below.

$$M + L \rightleftharpoons M\cdot L$$

The reaction is believed to be an equilibrium such that the concentrations of metal (M) and complexing agent (L) can affect the concentration of species present in solution. The equilibrium constant K (previously defined) is a mathematical expression that relates the concentrations of the reactants and reaction products involved in any equilibrium reaction. For chelation reactions this constant is called the stability constant and is a measure of the affinity of the complexing agent for a particular metal.

Competing side reactions, such as metal hydroxide formation, can also occur in aqueous solution.

$$xM + yOH^- \rightarrow M_x(OH)_y$$

The $OH^-$ concentration in solution which is related to pH is, therefore, an important parameter to be considered. If the pH is too high, the metal tends to form metal hydroxides rather than complexes. The complexing agents may also be affected by low pH. Complexation may require the loss of proton(s); therefore at low pH, conditions may not be favorable for complexation to occur. The complexes of this invention can be formed within a certain pH range. Consideration must be given to the solubility characteristics of the ligand, radionuclide, and complex. Although complexation will occur at other pH values, pH from 5 to 11 is preferred for complexation.

The metal and ligand may be combined under any conditions which allow the two to form a complex. Generally, mixing in water at a controlled pH (the choice of pH is dependent upon the choice of ligand and metal) is all that is required. In some cases heating may be required to obtain maximum complex yield.

The ratio of ligand to metal is a result of two competing considerations. As indicated above the ligand and metal are believed to be in equilibrium with the complex. On the other hand, it is desirable to have a large quantity of ligand (L), so that there is a minimum amount of free metal (M), because the uncomplexed metal may cause serious side effects in the patient. On the other hand, excess free ligand may be harmful and render the treatment less effective. Although it is difficult to generalize, it is believed that the required ligand to metal molar ratio is 1:1 to 1000:1.

The various complexes employed in this invention were prepared as follows: the desired amount of ligand was placed in a vial and dissolved by addition of water. At some higher ligand concentrations, it was necessary to add base in order to completely dissolve the ligand. Heating was also found to be useful for dissolving the ligands. The appropriate amount of the samarium or other radionuclides in the stock solution described above was then added to the ligand solution. The pH of the resulting solution was then raised to the appropriate level by addition of NaOH. The solution was heated to 60° to 70°C for 30 minutes in a water bath to insure maximum complex formation. The pH of the solution was then adjusted to 7 to 8 by addition of 1 M HCl.

The complex yield was determined by placing 5 to 20 microliters (depending on activity) of the complex solution onto a 0.5 cm³ column of a commercially available synthetic organic cation exchange resin. The column was then eluted with two separate 10 milliliter volumes of isotonic saline. The anionic complexes are not retained by the resin and were eluted by the saline solution, while any uncomplexed metal was retained on the column. The eluant solutions and the column were then counted for the characteristic emission of the particular radionuclide, e.g. 103 keV (16.5 × 10⁻¹⁵ joule) gamma ray of Sm-153. The complex yield was obtained by adding the counts in the eluant solutions and dividing by the total of the counts in the eluant solutions and the counts retained in the column.

This invention provides a means of delivering a therapeutic radiation dose to calcific tumors. It may also be desirable in the cases where the radionuclide has imageable gamma photons to inject a "sub-therapeutic" dose and determine the fate of the radionuclide using a scintillation camera prior to injecting a therapeutic radiation dose. Therefore, the levels of radiation injected could be as low as 1 mCi (37 MBq) for imaging prior to the therapy. Therapeutic doses will be greater. The dose to the tumor may range from 100 to 10,000 rads (1 Gy to 100 Gy). The preferred dose to the tumor ranges from 1,000 to 8,000 rads (10 Gy to 80 Gy). For complexes such as Sm-153-EDTMP amounts ranging from 0.1 mCi/kg (4 MBq/kg) body weight to 3 mCi/kg (11 MBq/kg) body weight are preferred. The amount of activity required to deliver a therapeutic dose may vary with the individual radionuclides. Individual doses may be given in one injection or fractionated into several injections totalling the aforementioned dose per treatment.

By fractionating the dose in administering the complex it may be possible to minimize damage to non-target tissue. Such multiple doses also may be more effective in alleviating pain and/or inhibiting tumor growth and/or causing regression of the tumor.

Studies to determine the qualitative distribution of the various complexes of the present invention were conducted by injecting the complexes into rats and obtaining the gamma ray images of the entire animal at various times up to two hours after injection.

Quantitative biodistributions were obtained by injecting 50 to 100 microliters of the complex solution into the tail vein of unanesthetized male Sprague Dawley rats. The rats were then placed in cages lined with absorbent paper in order to collect all urine excreted prior to sacrifice. After two hours, the rats were sacrificed by cervical dislocation and the various tissues dissected out. The samples were then rinsed with saline, blotted dry on absorbent paper and weighed. The radiation in the samples was measured with a NaI scintillation counter.

To minimize differences in age between groups of animals, all rats used were in the 160 to 220 gram weight range. All animals were kept "in-house" for at least one week prior to use to minimize the effects of stress that occur during shipping.

The following examples are illustrative of the present invention and are not to be construed as limiting the scope thereof.

## Example 1

25 to 35 milligrams of N-hydroxyethylethylene-diaminetriacetic acid (HEEDTA) was weighed into a vial and dissolved using 0.75 ml of distilled water. To this, 0.25 ml of Sm-153 (~ 10 mCi; ~370 MBq) in dilute HCl was added. The pH of the resulting solution was then adjusted to 10 by addition of NaOH. The resulting solution was heated to 60°C to 70°C for 30 minutes in a water bath to insure maximum complex formation. The pH of the solution was then adjusted to 7 to 8 by addition of 1 M HCl. The yield of the complex was over 95 percent.

A laboratory rat was injected with the above complex (50 to 100 μl) via the tail vein. After 2 hours, the animal was sacrificed by cervical dislocation and organs and tissues removed. Samples were counted with a NaI scintillation counter to determine the biolocalization of the complex. It was found that a significant amount (50 to 60 percent) of the activity was concentrated in the skeletal system with very little soft tissue uptake. Most of the activity not found in the skeleton was cleared through the kidneys into the bladder. Scintillation scans of animals treated in the same manner showed the activity concentrating in the skeletal system.

## Example 2

25 to 35 milligrams of N-hydroxyethylethylene-diaminetriacetic acid (HEEDTA) was weighed into a vial and dissolved using 0.75 ml of distilled water. To this, 0.25 ml of Gd-159 (~ 10 mCi; ~370 MBq) in dilute HCl

was added. The pH of the resulting solution was then adjusted to 10 by addition of NaOH. The resulting solution was heated to 60°C to 70°C for 30 minutes in a water bath to insure maximum complex formation. The pH of the solution was then adjusted to 7 to 8 by addition of 1 M HCl. The yield of the complex was over 95 percent.

Laboratory rats were injected with the above complex (50 to 100 µl) via the tail vein. After 2 hours, the animals were sacrificed by cervical dislocation and organs and tissues removed. Samples were counted with a NaI scintillation counter to determine the biolocalization of the complex. It was found that a significant amount (40—50 percent) of the activity was concentrated in the skeletal system with very little soft tissue uptake. Most of the activity not found in the skeleton was cleared through the kidneys into the bladder. Scintillation scans of animals treated in the same manner showed the activity concentrating in the skeletal system.

### Example 3

25 to 35 milligrams of N-hydroxyethylethylene-diaminetriacetic acid (HEEDTA) was weighed into a vial and dissolved using 0.75 ml of distilled water. To this, 0.25 ml of Yb-175 ($\sim$ 10 mCi; $\sim$ 370 MBq) in dilute HCl was added. The pH of the resulting solution was then adjusted to 10 by addition of NaOH. The resulting solution was heated to 60°C to 70°C for 30 minutes in a water bath to insure maximum complex formation. The pH of the solution was then adjusted to 7 to 8 by addition of 1 M HCl. The yield of the complex was over 95 percent.

Laboratory rats were injected with the above complex (50 to 100 µl) via the tail vein. After 2 hours, the animals were sacrificed by cervical dislocation and organs and tissues removed. Samples were counted with a NaI scintillation counter to determine the biolocalization of the complex. It was found that a significant amount (20 to 30 percent) of the activity was concentrated in the skeletal system with very little soft tissue uptake. Most of the activity not found in the skeleton was cleared through the kidneys into the bladder. Scintillation scans of animals treated in the same manner showed the activity concentrating in the skeletal system.

### Example 4

25 to 35 milligrams of N-hydroxyethylethylene-diaminetriacetic acid (HEEDTA) was weighed into a vial and dissolved using 0.75 ml of distilled water. To this, 0.25 ml of Lu-177 ($\sim$ 10 mCi; $\sim$370 MBq) in dilute HCl was added. The pH of the resulting solution was then adjusted to 10 by addition of NaOH. The resulting solution was heated to 60°C to 70°C for 30 minutes in a water bath to insure maximum complex formation. The pH of the solution was then adjusted to 7 to 8 by addition of 1 M HCl. The yield of the complex was over 95 percent.

Laboratory rats were injected with the above complex (50 to 100 µl) via the tail vein. After 2 hours, the animals were sacrificed by cervical dislocation and organs and tissues removed. Samples were counted with a NaI scintillation counter to determine the biolocalization of the complex. It was found that a significant amount (20 to 30 percent) of the activity was concentrated in the skeletal system with very little soft tissue uptake. Most of the activity not found in the skeleton was cleared through the kidneys into the bladder. Scintillation scans of animals treated in the same manner showed the activity concentrating in the skeletal system.

### Example 5

25 to 35 milligrams of disodium dihydrogen ethylenediaminetetraacetic acid dihydrate [$(Na)_2$EDTA·2 $H_2O$] was weighed into a vial and dissolved using 0.75 ml of distilled water. To this, 0.25 ml of Sm-153 ($\sim$ 10 mCi; $\sim$ 370 MBq) in dilute HCl was added. The pH of the resulting solution was then adjusted to 10 by addition of NaOH. The resulting solution was heated to 60°C to 70°C for 30 minutes in a water bath to insure maximum complex formation. The pH of the solution was then adjusted to 7 to 8 by addition of 1 M HCl. The yield of the complex was over 95 percent.

Laboratory rats were injected with the above complex (50 to 100 µl) via the tail vein. After 2 hours, the animals were sacrificed by cervical dislocation and organs and tissues removed. Samples were counted with a NaI scintillation counter to determine the biolocalization of the complex. It was found that a significant amount (40 to 50 percent) of the activity was concentrated in the skeletal system with very little soft tissue uptake. Most of the activity not found in the skeleton was cleared through the kidneys into the bladder. Scintillation scans of animals treated in the same manner showed the activity concentrating in the skeletal system.

The two-hour rat biodistribution data for the complexes of Examples 1 to 5 are given in Table 1.

EP 0 176 288 B1

TABLE 1
Example Nos.

| % Dose in | 1 | 2 | 3 | 4 | 5 |
|---|---|---|---|---|---|
| | Sm-153 HEEDTA | Gd-159 HEEDTA | Yb-175 HEEDTA | Lu-177 HEEDTA | Sm-153 EDTA |
| Bone | 58 | 44 ±4 | 25 ±2 | 21 ±2 | 43 ±6 |
| Blood | 0.18 | 0.13 ±0.11 | 0.33 ±0.12 | 0.40 ±0.19 | 0.38 ±0.03 |
| Liver | 2.42 | 0.73 ±0.09 | 0.36 ±0.04 | 2.62 ±0.98 | 4.4 ±1.4 |
| Bone/Blood | 245 | 297 ±78 | 64 ±21 | 50 ±22 | 84 ±12 |
| Bone/Muscle | 426 | 649 ±182 | 199 ±60 | 97 ±22 | 184 ±27 |

Comparative Examples A and B

Samarium-153 complexes with (A) nitrilotriacetic acid (NTA), Log K = 11.3, and (B) diethylenetriaminepentaacetic acid (DTPA), Log K = 22.3, were tested in the manner of Example 1. Results are given in Table 2.

TABLE 2

| % Dose in | Example A | Example B |
|---|---|---|
| Bone | 49 ±3 | 0.19 ±0.09 |
| Blood | 0.64 ±0.27 | 0.19 ±0.05 |
| Liver | 8.6 ±1.8 | 0.29 ±0.12 |
| Bone/Blood | 86 ±50 | 0.8 ±0.6 |
| Bone/Muscle | 171 ±106 | 1.0 ±0.5 |

It should be noted that the comparative examples have certain undesirable properties which make them inferior to the complexes of the invention. Thus, the NTA complexed with Sm-153 shows high liver uptake while the DTPA complex has low skeletal uptake.

The Log K values for the complexes of Examples 1 to 5 are as follows:—

| Example | Log K |
|---|---|
| 1 | 15.4 |
| 2 | 15.3 |
| 3 | 16.0 |
| 4 | 16.0 |
| 5 | 17.1 |

8

# EP 0 176 288 B1

**Claims**

1. Use for the manufacture of a medicament for the treatment of calcific tumors of a complex of Gadolinium-159 (Gd-159), Holmium-166 (Ho-666), Lutetium-177 (Lu-177), Samarium-153 (Sm-153) or Ytterbium-175 (Yb-175) with a chelant selected from organic aminocarboxylic acid derivatives having at least one of the amine hydrogens replaced by a carboxyalkyl radical and physiologically acceptable salts thereof, wherein the molar ratio of chelant to metal is above 10:1 and the log of the stability constant (Log K) is from 13 to 20.

2. A use as claimed in Claim 1, wherein said chelant an ethylenediaminecarboxylic acid or a physiologically acceptable salt thereof.

3. A use as claimed in Claim 2, wherein said ethylenediaminecarboxylic acid is an ethylenediamineacetic acid.

4. A use as claimed in Claim 3, wherein said ethylenediamineacetic acid is ethylenediaminetetraacetic acid.

5. A use as claimed in Claim 4, wherein the radionuclide is Sm-153.

6. A use as claimed in Claim 3, wherein at least one of the amine hydrogens of said ethylenediamineacetic acid is replaced by a hydroxyalkyl radical containing 2 or 3 carbon atoms.

7. A use as claimed in Claim 6, wherein said ethylenediamineacetic acid is hydroxyethylethylenediaminetriacetic acid.

8. A use as claimed in Claim 7, wherein the radionuclide is Sm-153.

9. A use as claimed in Claim 7, wherein the radionuclide is Gd-159.

10. A use as claimed in Claim 7, wherein the radionuclide is Ho-166.

11. A use as claimed in Claim 7, wherein the radionuclide is Lu-177.

12. A use as claimed in Claim 7, wherein the radionuclilde is Yb-175.

13. A use as claimed in any one of the preceding claims, wherein the said aminocarboxylic acid derivative is in the form of a physiologically acceptable salt of an alkali metal.

14. A use as claimed in Claim 13, wherein the alkali metal is sodium or potassium.

15. A use as claimed in any one of the preceding claims, wherein said complex is obtainable by contacting the rare earth radionuclide with the chelant in the presence of water at a pH from 5 to 11, in a ratio of 10 mCi metal to 25 to 35 mg chelant.

16. A use as claimed in any one of the preceding claims, wherein said molar ratio does not exceed 1000.1.

17. A use as claimed in any one of the preceding claims, wherein the chelant to metal molar ratio is sufficiently large that the bone/liver biodistribution in the rat is at least 8.

18. A therapeutically effective complex of Gadolinium-159 (Gd-159), Holmium-166 (Ho-666), Lutetium-177 (Lu-177), Samarium-153 (Sm-153) or Ytterbium-175 (Yb-175) with a chelant selected from ethylenediamineacetic acids in which at least one of the amine hydrogens is replaced by a hydroxyalkyl radical containing 2 or 3 carbon atoms and physiologically acceptable salts thereof, wherein the log of the stability constant (Log K) is from 13 to 20.

19. A complex as claimed in Claim 18, wherein said ethylenediamineacetic acid is hydroxyethylethylenediaminetriacetic acid.

20. A complex as claimed in Claim 18 or Claim 19, wherein the said aminocarboxylic acid derivative is in the form of a physiologically acceptable salt of an alkali metal.

21. A complex as claimed in Claim 20, wherein the alkali metal is sodium or potassium.

22. A complex as claimed in any one of Claims 18 to 21, wherein the chelant to radionuclide molar ratio is from 1:1 to 1000:1.

23. A complex as claimed in any one of Claims 18 to 22, wherein the chelant to radionuclide molar ratio is sufficiently large that the bone/liver biodistribution in the rat is at least 8.

24. A composition for the treatment of calcific tumors comprising a complex as claimed in any one of Claims 18 to 23 with a physiologically acceptable carrier or diluent therefor.

25. A composition as claimed in Claim 24 in unit dosage form, wherein each unit dose contains a therapeutic amount of said complex, said amount exceeding that required for use as a diagnostic skeletal agent.

26. A complex as defined in any one of Claims 18 to 23, for use in the treatment of calcific tumors.

27. The use of a complex as defined in any one of Claims 18 to 23 in the manufacture of a medicament for the treatment of calcific tumors.

**Patentansprüche**

1. Verwendung eines Komplexes aus Gadolinium-159 (Gd-159), Holmium-166 (Ho-666), Lutetium-177 (Lu-177), Samarium-153 (Sm-153) oder Ytterbium-175 (Tb-175) mit einem Chelatbildner, ausgewählt aus organischen Aminocarbonsäurederivaten, in denen mindestens eines der Amin-Wasserstoffatome durch ein Carboxyalkyl-Radikal ersetzt ist, und physiologisch anehmbaren Salzen davon, wobei das molare Verhältnis von Chelatbildner zu Metall größer als 10:1 ist, und der log der Stabilitätskonstante (Log K) 13 bis 20 beträgt, zur Herstellung eines Medikamentes zu Behandlung kalkbildender Tumore.

2. Verwendung nach Anspruch 1, dadurch gekennzeichnet, daß der Chelatbildner eine Ethylendiamincarbonsäure oder ein physiologisch annehmbares Salz davon ist.

3. Verwendung nach Anspruch 2, dadurch gekennzeichnet, daß die Ethylendiamincarbonsäure Ethylendiaminessigsäure ist.

4. Verwendung nach Anspruch 3, dadurch gekennzeichnet, daß die Ethylendiaminessigsäure Ethylendiamintetraessigsäure ist.

5. Verwendung nach Anspruch 4, dadurch gekennzeichnet, daß das Radionuklid Sm-153 ist.

6. Verwendung nach Anspruch 3, dadurch gekennzeichnet, daß mindestens eines der Amin-Wasserstoffatome der Ethylendiaminessigsäure durch ein Hydroxyalkylradikal mit 2 oder 3 Kohlenstoffatomen ersetzt ist.

7. Verwendung nach Anspruch 6, dadurch gekennzeichnet, daß die Ethylendiaminessigsäure Hydroxyethylethylendiamintriessigsäure ist.

8. Verwendung nach Anspruch 7, dadurch gekennzeichnet, daß das Radionuklid Sm-153 ist.

9. Verwendung nach Anspruch 7, dadurch gekennzeichnet, daß das Radionuklid Gd-159 ist.

10. Verwendung nach Anspruch 7, dadurch gekennzeichnet, daß das Radionuklid Ho-166 ist.

11. Verwendung nach Anspruch 7, dadurch gekennzeichnet, daß das Radionuklid Lu-177 ist.

12. Verwendung nach Anspruch 7, dadurch gekennzeichnet, daß das Radionuklid Yb-175 ist.

13. Verwendung nach einem der vorhergehenden Ansprüche, dadurch gekennzeichnet, daß das Aminocarbonsäure-Derivat in Form eines physiologisch annehmbaren Salzes eines Alkalimetalles vorliegt.

14. Verwendung nach Anspruch 13, dadurch gekennzeichnet, daß das Alkalimetall Natrium oder Kalium ist.

15. Verwendung nach einem der vorhergehenden Ansprüche, dadurch gekennzeichnet, daß der Komplex erhältlich ist durch Inkontaktbringen des Seltenerd-Radionuklid mit dem Chelatbildner in Gegenwart von Wasser bei einem pH-Wert von 5 bis 11 in einem Verhältnis von 10 mCi Metall zu 25 bis 35 mg Chelatbildner.

16. Verwendung nach einem der vorhergehenden Ansprüche, dadurch gekennzeichnet, daß das molare Verhältnis 1000:1 nicht übersteigt.

17. Verwendung nach einem der vorhergehenden Ansprüche, dadurch gekennzeichnet, daß das molare Verhältnis von Chelatbildner zu Metall ausreichend groß ist, sodaß die Knochen/Leber-Bioverteilung in der Ratte mindestens 8 ist.

18. Therapeutisch wirksamer Komplex von Gadolinium-159 (Gd-159), Holmium-166 (Ho-166), Lutetium-177 (Lu-177), Samarium-153 (Sm-153) oder Ytterbium-175 (Yb-175) mit einem Chelatbildner, ausgewählt aus Ethylendiaminessigsäuren, in denen mindestens eines der Amin-Wasserstoffe durch ein Hydroxyalkyl-Radikal mit zwei oder drei Kohlenstoffatomen ersetzt ist, und physiologisch annehmbaren Salzen davon, worin der log der Stabilitätskonstante (Log K) 13 bis 20 ist.

19. Komplex nach Anspruch 18, dadurch gekennzeichnet, daß die Ethylendiaminessigsäure Hydroxyethylethylendiamintriessigsäure ist.

20. Komplex nach Anspruch 18 oder 19, dadurch gekennzeichnet, daß das Aminocarbonsäure-Derivat in Form eines physiologisch annehmbaren Salzes eines Alkalimetalls vorliegt.

21. Komplex nach Anspruch 20, dadurch gekennzeichnet, daß das Alkalimetall Natrium oder Kalium ist.

22. Komplex nach einem der Ansprüche 18 bis 21, dadurch gekennzeichnet, daß das molare Verhältnis von Chelatbildner zu Radionuklid 1:1 bis 1000:1 ist.

23. Komplex nach einem der Ansprüche 18 bis 22, dadurch gekennzeichnet, daß das molare Verhältnis von Chelatbildner zu Radionuklid ausreichend groß ist, sodaß die Knochen/Leber-Bioverteilung in Rate in mindestens 8 beträgt.

24. Zusammensetzung zur Behandlung kalkbildender Tumore, umfassend einen Komplex nach einem der Ansprüche 18 bis 23 zusammen mit einem physiologisch annehmbaren Träger oder Verdünner dafür.

25. Zusammensetzung nach Anspruch 24 in Form einer Dosiereinheit, dadurch gekennzeichnet, daß jede Dosiereinheit eine therapeutische Menge des Komplexes enthält, wobei diese Menge diejenige übersteigt, die zu Verwendung als Skelett-Diagnostikum erforderlich ist.

26. Komplex nach einem der Ansprüche 18 bis 23 zur Verwendung bei der Behandlung kalkbildender Tumore.

27. Verwendung eines Komplexes nach einem der Ansprüche 18 bis 23 zur Herstellung eines Medikamentes zur Behandlung kalkbildender Tumore.

**Revendications**

1. Utilisation, pour la fabrication d'un médicament pour le traitement du tumeurs calcifiantes, d'un complex du gadolinium-159 (Gd-159), du holmium-166 (Ho-166), du lutétium-177 (Lu-177), du samarium-153 (Sm-153) ou de l'ytterbium-175 (Yb-175) avec un agent chélatant choisi parmi les dérivés organiques acides aminocarboxyliques dans lesquels au moins un des hydrogènes de l'amine est remplacé par un radical carboxyalkyle et leurs sels physiologiquement acceptables, le rapport molaire de l'agent chélatant au métal étant supérieur à 10:1, et le log de la constante de stabilité (Log K) valant de 13 à 20.

2. Utilisation selon la revendication 1, dans laquelle ledit agent chélatant est un acide éthylènediaminecarboxylique ou un de ses sels physiologiquement acceptables.

3. Utilisation selon la revendication 2, dans laquelle ledit acide éthylénediaminecarboxylique est un acide éthylènediamineacétique.

4. Utilisation selon la revendication 3, dans laquelle ledit acide éthylènediamineacétique est l'acide éthylédiaminetétraacétique.

5. Utilisation selon la revendication 4, dans laquelle le radionucléide est le Sm-153.

6. Utilisation selon la revendication 3, dans laquelle au moins un des hydrogènes de l'amine dudit acide éthylédiamineacétique est remplacé par un radical hydroxyalkyle contenant 2 ou 3 atomes de carbone.

7. Utilisation selon la revendication 6, dans laquelle ledit acide éthylènediamineacétique est l'acide hydroxyéthyl-éthylénediaminetriacétique.

8. Utilisation selon la revendication 7, dans laquelle le radionucléide est le Sm-153.

9. Utilisation selon la revendication 7, dans laquelle le radionucléide est le Gd-159.

10. Utilisation selon la revendication 7, dans laquelle le radionucléide est le Ho-166.

11. Utilisation selon la revendication 7, dans laquelle le radionucléide est le Lu-177.

12. Utilisation selon la revendication 7, dans laquelle le radionucléide est le Yb-175.

13. Utilisation selon l'une quelconque des revendications précédentes, dans laquelle ledit dérivé acide aminocarboxylique est sous la forme d'un sel physiologiquement acceptable d'un métal alcalin.

14. Utilisation selon la revendication 13, dans laquelle le métal alcalin est le sodium ou le potassium.

15. Utilisation selon l'une quelconque des revendications précédentes, dans laquelle ledit complexe peut être obtenu par mise en contact du radionucléide de terre rare avec l'agent chélatant, en présence d'eau, à un pH de 5 à 11, dans un rapport de 10 mCi de métal pour 25 à 35 mg d'agent chélatant.

16. Utilisation selon l'une quelconque des revendications précédentes, dans laquelle ledit rapport molaire ne dépasse pas 1000:1.

17. Utilisation selon l'une quelconque des revendications précédentes, dans laquelle le rapport molaire de l'agent chélatant au métal est suffisamment grand pour que la biodistribution os/foie chez le rat soit d'au moins 8.

18. Complexe thérapeutiquement efficace du gadolinium-159 (Gd-159), du holmium-166 (Ho-166), du lutétium-177 (Lu-177), du samarium-153 (Sm-153) ou de l'ytterbium-175 (Yb-175) avec un agent chélatant choisi parmi les acides éthylènediamineacétiques dans lesquels au moins un des hydrogènes de l'amine est remplacé par un radical hydroxyalkyle contenant 2 ou 3 atomes de carbone et leurs sels physiologiquement acceptables, et dont le log de la constante de stabilité (Log K) vaut de 13 à 20.

19. Complexe selon la revendication 18, dans lequel ledit acide éthylènediamineacétique est l'acide hydroxyéthyl-éthylénediaminetriacétique.

20. Complexe selon la revendication 18 ou 19, dans laquelle ledit dérivé acide aminocarboxylique est sous la forme d'un sel physiologiquement acceptable d'un métal alcalin.

21. Complexe selon la revendication 20, dans lequel le métal alcalin est le sodium ou le potassium.

22. Complexe selon l'une quelconque des revendications 18 à 21, dans lequel le rapport molaire de l'agent chélatant au radionucléide vaut de 1:1 à 1000:1.

23. Complexe selon l'une quelconque des revendications 18 à 22, dans lequel le rapport molaire de l'agent chélatant au radionucléide est suffisamment grand pour que la biodistribution os/foie chez le rat soit d'au moins 8.

24. Composition pour le traitement du tumeurs calcifiantes comprenant un complexe selon l'une quelconque des revendications 18 à 23 avec un support ou diluant physiologiquement acceptable de celui-ci.

25. Composition selon la revendication 24, présentée en doses unitaires, chaque dose unitaire contenant une quantité thérapeutique dudit complexe, ladite quantité étant supérieure à celle nécessaire pour l'utilisation comme agent de diagnostic pour le squelette.

26. Complexe selon l'une quelconque des revendications 18 à 23, pour l'utilisation dans le traitement des tumeurs calcifiantes.

27. Utilisation d'un complexe selon l'une quelconque des revendications 18 à 23, dans la fabrication d'un médicament pour le traitement des tumeurs calcifiantes.